# EUROPEAN PATENT SPECIFICATION

(11) **EP 4 529 877 B1**
(45) Date of publication and mention of the grant of the patent: **28.10.2026**
(21) Application number: 24200761.5
(22) Date of filing: 17.09.2024
(51) Int. Cl.: A61B 18/18

(54) **CONSOLE ARCHITECTURE FOR MICROWAVE ABLATION UNIT**
KONSOLENARCHITEKTUR FÜR MIKROWELLENABLATIONSEINHEIT
ARCHITECTURE DE CONSOLE POUR UNITÉ D'ABLATION PAR MICRO-ONDES

(30) Priority: 28.09.2023 US 202318477347
(43) Date of publication of application: 02.04.2025
(73) Proprietor: Varian Medical Systems, Inc., Palo Alto, CA 94304 (US)
(72) Inventor: JESTER, Steve, Eden Prairie, 55346 (US); OLSON, Dane, New Hope, 55427 (US); HATTAN, Paul, Minneapolis, 55401 (US); BACHMAN, Tim, St. Paul, 55116 (US); GOULET, Matt, St. Paul, 55104 (US); WEBER, Charlie, St Paul, 55104 (US); ERNSTER, Logan, St. Paul, 55012 (US)
(74) Representative: Mathisen & Macara LLP

(56) References cited:
- WO-A1-2013/008843
- CN-U- 213 755 025
- CN-U- 216 134 177

## Description

### FIELD

The present disclosure relates to console architecture for a microwave ablation console. More particularly, the present disclosure relates to the structure and features of a microwave ablation console that may include a cooling structure for managing thermal energy generated by the components of the microwave ablation console.

### BACKGROUND

This section provides background information related to the present disclosure which is not necessarily prior art.

Ablation apparatuses and systems are used to perform ablation treatments in which a probe is inserted at or near a target tissue in a patient. The target tissue is typically an abnormal tissue such as a tumor or other growth. The ablation treatment is performed to destroy the target tissue to reduce the likelihood of further growth or spreading of the target tissue in the patient. One type of ablation is thermal ablation. In thermal ablation, the probe that is positioned at or near the target tissue causes the temperature of the target tissue, typically in a localized region proximate the probe, to be elevated to a temperature at which the target tissue is destroyed.

The thermal ablation can be induced by the emission of microwaves at or near the target tissue. A microwave generator may be used that sends a microwave signal to an antenna in the ablation probe. The antenna may emit microwave energy into surrounding tissue causing the temperature of such tissue to be elevated to temperatures sufficient to cause thermal ablation.

Existing microwave ablation systems and apparatuses suffer from various drawbacks. For example, existing ablation systems can be large, cumbersome and difficult to use. The complexity and structure of existing systems may require extensive training and may be highly dependent on the experience level of the operator. Existing ablation apparatuses may also be poor at managing the temperatures that various elements of the microwave ablation systems may generate during operation. Elevated temperatures can lead to premature failure of components or may not permit functionality that may otherwise be desired. There exists a need, therefore, for improved ablation apparatuses and systems that can be easily operated and reduce the likelihood of premature failure and/or detrimental effects of elevated temperatures.

### SUMMARY

This section provides a general summary of the disclosure, and is not a comprehensive disclosure of its full scope or all of its features.

The invention is defined in the appended set of claims. The present disclosure provides various aspects of a microwave ablation console. The console may be configured to provide improved durability and reliability over existing microwave ablation consoles. Furthermore, the consoles of the present disclosure can be portable and be easily used in various locations in a treatment environment.

The present invention provides an apparatus for generating a microwave signal which includes a housing comprising a plurality of walls defining an interior volume, an inlet opening and an outlet opening; a power supply positioned in the interior volume; a first microwave generator and a second microwave generator each operably coupled to the power supply and each positioned in the interior volume spaced apart from the power supply; and a heat sink positioned in the interior volume and contacting the power supply, the first microwave generator and the second microwave generator; and a fan positioned in the interior volume at a first end of the heat sink, the fan configured to draw an airflow into the interior volume from the inlet opening and move the airflow through the heat sink.

In one aspect, the fan may be offset from an inlet duct that is coupled to the inlet opening.

In another aspect, the first generator and the second generator may be positioned on opposite sides of the heat sink.

In another aspect, the heat sink may include a plurality of walls defining a chamber and a plurality of fins positioned in the chamber.

In another aspect, the apparatus may include an outlet duct coupled to a second end of the heat sink downstream of the first end, wherein heated airflow from the heat sink is moved out the outlet opening through the inlet duct.

In another aspect, the second end may be located at a position opposite to the first end.

In another aspect, the apparatus may also include a plurality of coolant pumps positioned in the interior volume spaced apart from the heat sink.

In another aspect, at least a portion of the airflow that enters the interior volume through the inlet opening contacts internal components in the interior volume before entering the heat sink.

In another aspect, the apparatus may include an inlet duct coupled to the inlet opening. The inlet duct may guide incoming air upwards into the interior volume.

In another aspect, the housing may include a first shell portion and a second shell portion. The first shell portion may have a lip positioned around an outer circumference that is configured to overlap an outer circumference of the second shell portion.

In another aspect, the housing may include a first handle that projects inward from an outer surface of the housing and the inlet opening may be positioned in the first handle.

In another aspect, the housing may include a second handle that projects inward from the outer surface of the housing and the outlet opening may be positioned in the second handle.

In another aspect, the first handle and the second handle may be positioned on opposite sides of the housing.

In another aspect, the housing may include a hinge configured to support a display.

The apparatus comprises a hinge configured to allow angular and rotational movement of the display relative to the housing.

In another aspect, the apparatus may include a stand configured to support a coolant receptacle in a predetermined position relative to the housing.

In another aspect, the first microwave generator and the second microwave generator may be both powered by the power supply.

In another aspect, the first microwave generator and the second microwave generator may be independently operable to deliver a first microwave signal and a second microwave signal, respectively.

In another aspect, the first microwave signal and the second microwave signal are different.

In another aspect, the power supply comprises a single power input.

In another aspect, the present invention provides a housing for enclosing a microwave ablation unit, the housing comprising a first shell portion, a second shell portion comprising a lip for mating to the first shell portion and a contoured upper surface for guiding fluids toward one or more sides; and a face portion configured to mate to a front opening defined by the first shell portion and the second shell portion.

In one aspect, the housing may include a back plate configured to mate to a back opening defined by the first shell portion and the second shell portion.

In another aspect, the first shell portion may include an inlet opening and an outlet opening. The inlet opening may be configured to accept an incoming airflow and the outlet opening configured to dispense an outgoing airflow.

In another aspect, the inlet opening is positioned in an inset handle formation.

In another aspect, the face portion may include a first microwave port and a second microwave port each configured to accept a connector of a microwave ablation probe assembly.

In another aspect, the face portion may include a first coolant port and a second coolant port. The first coolant port and the second coolant port may each be configured to accept a coolant cartridge of a microwave ablation probe assembly.

In another aspect, the contoured upper surface is configured to guide fluids away from the face portion.

In another aspect, the lip is configured to overlap a complimentary lip of the first shell portion.

In another aspect, the first shell portion, the second shell portion, and the face portion may define an interior volume configured to retain a microwave generator, a power supply, a heat sink, and a coolant pump.

In another aspect, the microwave generator is a first microwave generator, the coolant pump is a first coolant pump, and the interior volume may be configured to retain a second microwave generator and a second coolant pump.

In another aspect, the present invention provides a cooling structure for managing thermal energy in a microwave ablation console, the cooling structure comprising a heat sink comprising: a first wall including one or more first connection points for retaining a first microwave generator, a second wall including one or more second connection points for retaining a second microwave generator, a third wall including one or more third connection points for retaining a power supply, and a fourth wall, wherein the first wall, the second wall, the third wall, and the fourth wall define a chamber; an inlet manifold for guiding air into the chamber; an outlet manifold for guiding the air out of the chamber, and a fan connected to the inlet manifold.

In one aspect, the first wall and the second wall may be positioned on opposite sides of the heat sink.

In another aspect, the heat sink may include a plurality of fins positioned inside the chamber.

In another aspect, the first wall, the second wall, the third wall, and the fourth wall may be connected to form a rectangular shape.

Further areas of applicability will become apparent from the description provided herein. The description and specific examples in this summary are intended for purposes of illustration only and are not intended to limit the scope of the present disclosure.

### DRAWINGS

The drawings described herein are for illustrative purposes only of selected embodiments and not all possible implementations, and are not intended to limit the scope of the present disclosure.
FIG. 1 is an isometric view of an example microwave ablation system in accordance with some embodiments of the present disclosure.
FIG. 2 is an isometric view of an example microwave ablation console that can be part of the microwave ablation system of FIG. 1.
FIG. 3 is another isometric view of the example microwave ablation console of FIG. 2.
FIG. 4 is bottom view of the microwave ablation console of FIG. 3.
FIG. 5 is a left side bottom view of the microwave ablation console of FIG. 3.
FIG. 6 is another isometric view of the example microwave ablation console of FIG. 3 shown with the housing transparent in order to view internal components.
FIG. 7 is a rear isometric view of the console of FIG. 6 also shown with the housing transparent.
FIG. 8 is a bottom view of the console of FIG. 6 shown with the housing transparent.
FIG. 9 is a front view of the console of FIG. 6 shown with the housing transparent.
FIG. 10 is a top view of the console of FIG. 6 shown with the upper shell portion removed to show internal components.
FIG. 11 is magnified view of the console of FIG. 10 showing an example inlet duct and fan.
FIG. 12 is another magnified view of the console of FIG. 10 showing the example inlet duct, fan, transition, and heat sink.

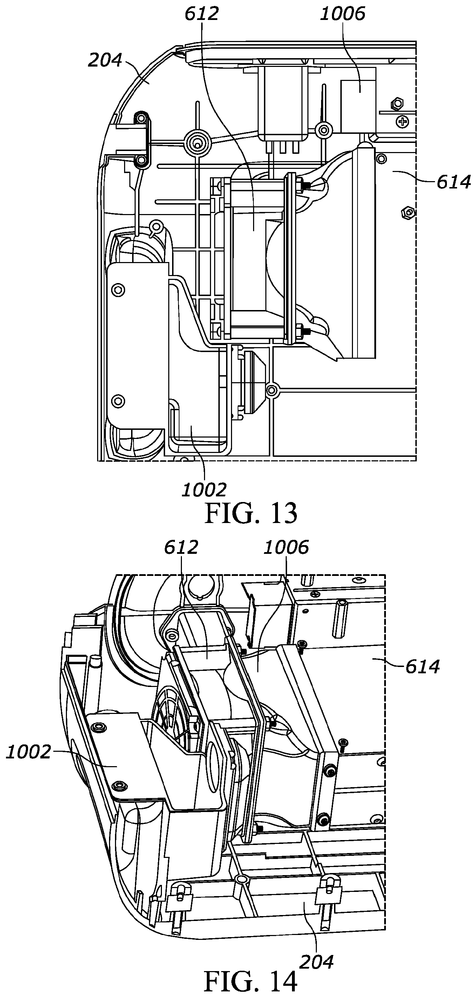

FIG. 13 is another magnified view showing a top view of the inlet duct, fan, transition and heat sink.
FIG. 14 is another magnified view showing the inlet duct, fan, transition and heat sink.
FIG. 15 is a magnified view showing an example outlet duct and heat sink.
FIG. 16 is a magnified view showing a top view of the outlet duct and heat sink of FIG. 15.
FIG. 17 is an isometric view showing another example housing including another example heat sink in accordance with some embodiments of the present disclosure.
FIG. 18 is a rear isometric view of the example housing and heat sink of FIG. 17.
FIG. 19 is a side view of the housing and heat sink of FIG. 17.
FIG. 20 is a front view of the housing and heat sink of FIG. 17.
FIG. 21 is a isometric view of the example sink of FIG. 17 shown with the walls of the heat sink transparent to show the fins in the internal chamber.

Corresponding reference numerals indicate corresponding parts throughout the several views of the drawings.

### DETAILED DESCRIPTION

Example embodiments will now be described more fully with reference to the accompanying drawings. For purposes of the description hereinafter, it is to be understood that the embodiments described below may assume alternative variations and embodiments. It is also to be understood that the specific articles, compositions, and/or processes described herein are exemplary and should not be considered as limiting. In the description, relative terms such as "lower," "upper," "horizontal," "vertical,", "above," "below," "up," "down," "top" and "bottom" as well as derivative thereof (e.g., "horizontally," "downwardly," "upwardly," etc.) should be construed to refer to the orientation as then described or as shown in the drawing under discussion. These relative terms are for convenience of description and do not require that the apparatus be constructed or operated in a particular orientation. Terms concerning attachments, coupling and the like, such as "connected" and "interconnected," refer to a relationship wherein structures are secured or attached to one another either directly or indirectly through intervening structures, as well as both movable or rigid attachments or relationships, unless expressly described otherwise.

Although the terms first, second, third, etc. may be used herein to describe various elements, components, regions, layers and/or sections, these elements, components, regions, layers and/or sections should not be limited by these terms. These terms may be only used to distinguish one element, component, region, layer or section from another region, layer or section. Terms such as "first," "second," and other numerical terms when used herein do not imply a sequence or order unless clearly indicated by the context. Thus, a first element, component, region, layer or section discussed below could be termed a second element, component, region, layer or section without departing from the teachings of the example embodiments.

In the present disclosure the singular forms "a," "an," and "the" include the plural reference, and reference to a particular numerical value includes at least that particular value, unless the context clearly indicates otherwise. When values are expressed as approximations, by use of the antecedent "about," it will be understood that the particular value forms another embodiment. As used herein, "about X" (where X is a numerical value) preferably refers to ±10% of the recited value, inclusive. For example, the phrase "about 8" preferably refers to a value of 7.2 to 8.8, inclusive. Where present, all ranges are inclusive and combinable. For example, when a range of "1 to 5" is recited, the recited range should be construed as including ranges "1 to 4", "1 to 3", "1-2", "1-2 & 4-5", "1-3 & 5", "2-5", and the like. In addition, when a list of alternatives is positively provided, such listing can be interpreted to mean that any of the alternatives may be excluded, e.g., by a negative limitation in the claims. For example, when a range of "1 to 5" is recited, the recited range may be construed as including situations whereby any of 1, 2, 3, 4, or 5 are negatively excluded; thus, a recitation of "1 to 5" may be construed as "1 and 3-5, but not 2", or simply "wherein 2 is not included." It is intended that any component, element, attribute, or step that is positively recited herein may be explicitly excluded in the claims, whether such components, elements, attributes, or steps are listed as alternatives or whether they are recited in isolation.

In some embodiments of the present disclosure, an improved microwave ablation console is provided. The improved microwave ablation console may overcome various drawbacks of existing consoles and also improve performance, versatility and durability over known systems and methods. In some embodiments, the microwave ablation consoles of the present disclosure may include an improved housing that can include features, shape, and structure to allow improved functionality. The housings of the present disclosure may allow portability of the microwave ablation console. The housings may also reduce a likelihood of damage due to spills that may occur in a treatment environment. Still further, the housing may be configured to retain various components in desired positions and allow thermal management to be performed to reduce operating temperatures of the various internal components.

The microwave ablation consoles of the present disclosure may also include improved features that may include adjustable display screens and various ports and/or connectors to allow a simplified set-up and operation over that of existing systems. The microwave ablation consoles of the present disclosure may require less time to set-up and operate over existing systems. In addition, the simplified and intuitive structure may reduce a likelihood that an error may be made during set-up and operation. These improved may lead to improved patient outcomes and a reduction in treatment durations.

The microwave ablation consoles of the present disclosure may also include multiple microwave generators in a single integrated unit. Such functionality can allow multiple independent ablation probes to be used during a treatment and be controlled using single console. This functionality coupled with the portability of the console is an improvement over existing systems that are not portable and may not allow independent microwave generation in a small, compact unit. In addition to multiple microwave generators, the consoles of the present disclosure may also include multiple coolant pumps. The multiple coolant pumps may be configured to cause coolant to be moved through cooling lines to cool the cables of the ablation probe assemblies. This is also an improvement over existing consoles and systems that often required separate coolant pump assemblies that are not integrated into a portable microwave ablation console.

Existing microwave ablation systems often do not include multiple microwave generators because it can be difficult to manage the thermal energy produced by the microwave generators during operation. The microwave generator can generate enough heat to damage the generator itself or other components in the microwave ablation console if the heat is not sufficiently managed. The microwave ablation consoles of the present disclosure can include an improved heat sink that can move thermal energy away from the microwave generators and the relevant power supply in order to maintain appropriate operating temperatures for the components in the console.

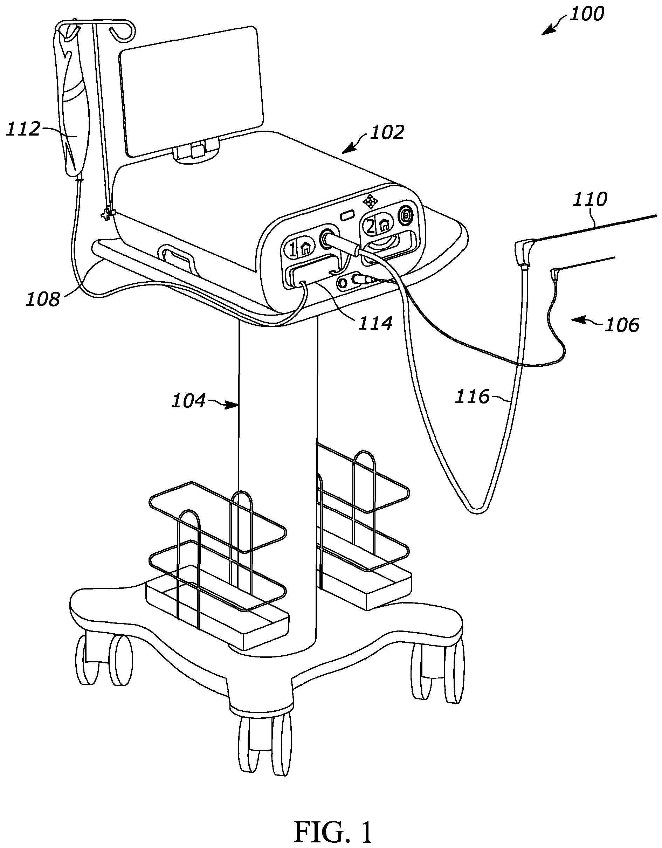

Referring now to FIG. 1, an example microwave ablation apparatus 100 is shown. The microwave ablation apparatus 100 may include a portable base unit or console 102, a cart 104, and an ablation probe assembly 106. The console 102 can be positioned on the cart 104. As will be further described, the console 102 may include a microwave generator and a coolant pump. In various embodiments, the ablation apparatus 100 can be operated to perform ablation treatments by sending a microwave signal to an antenna in an ablation probe 110 via cable 116. The antenna may deliver the microwaves to a target tissue at or near the ablation probe 110. The microwaves may induce an elevated temperature at the target tissue to destroy the target tissue.

During the ablation treatment, the ablation apparatus 100 may also deliver a coolant from a coolant receptacle 112 to the probe 110 using the coolant flow generator. The coolant cartridge 114 may be inserted into the front face of the console 102 and engage a pump to move the coolant from the coolant receptacle 112 to the probe 110 through the cable 116. The movement of the coolant may absorb thermal energy from the cable and/or probe 110 effectively cooling the cable and/or portions of the probe 110.

As can be seen, the console 102 and the cart 104 can be easily moved in a hospital, treatment center, or other environment. The cart 104 may include a support surface such as platform 108 that is positioned at an elevated position above the floor to allow access to the functionality of the system. The platform 108 may be positioned, for example, at a height approximately the same as a height of a patient. The patient that is undergoing an ablation treatment may be, for example, positioned on a bed of an imaging system. The imaging system may be a CT scanner, MRI machine, X-ray apparatus or other imaging device. With the console 102 positioned at a height approximately equal to that of the bed of the imaging system, the ablation treatment can be more easily performed. In addition, the console 102 can be moved next to the bed so that the length of the cables 116 that may be required to deliver microwave signals and/or coolant from the base unit to the probe 110 can be reduced over ablation systems that are more cumbersome or may need to be located at other positioned in the treatment environment.

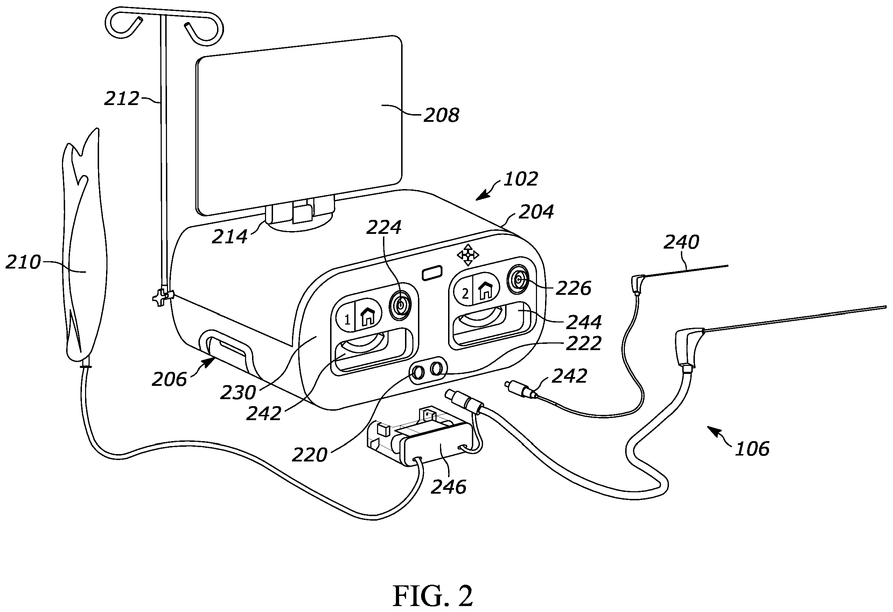

The console 102 can be portable in that it can be lifted and moved by one user. The console 102 can be removed from the cart 104 and be positioned on a table, counter, or other surface in a treatment or laboratory setting. The console 102 may include a housing 204 that is positioned around the inner components of the console 102. The housing 204 may include one or more walls that define an interior volume in which the inner components are located. As shown in FIG. 2, the console 102 may include a first microwave port 224, a second microwave port 226, a first sensor port 220, and a second sensor port 222. The console 102 may also include a display 208 to allow input from a user and to display one or more operating parameters of the console 102.

The first microwave port 224 and the second microwave port 226 can be similarly configured and can be an electrical connector to allow a mating connector to electrically couple the ablation probe assembly 106 to a respective microwave generator positioned inside the housing 204. In some examples, the first microwave port 224 may include a first marking and the second microwave port 226 may include a second marking. The first marking and the second marking may be different to allow a user to differentiate between the ports. The first marking and the second marking may include various suitable identifiers such as a color, a symbol, a letter, a number, a shape, a colored light or others. In some examples, the first marking and the second marking may include multiple identifiers from the previous list of identifiers. The first marking and the second marking can be used so as to guide an operator in the set-up and use of the console 102 to ensure that the proper connections are made by the operator.

The first microwave port 224 and the second microwave port 226 may be positioned in a front face 230 of the housing 204. This may allow a user to easily connect an ablation probe assembly 106 to the first microwave port 224 and/or the second microwave port 226. In other examples, the first microwave port 224 and the second microwave port 226 may be positioned at other locations in the housing 204 such as in the side walls, or top surface of the housing 204. While the example console 102 shown in FIG. 2 includes two microwave generators and two microwave ports, it should be appreciated that one generator and port may be included in some examples and more than two generators and ports may be included in other examples.

As further shown in this example, the console 102 may include a first sensor port 220 and a first sensor port 222 positioned on the front face 230. This positioning can allow an operator to easily connect one or more sensors to the console 102. As shown in FIG. 2, the ablation probe assembly 106 may include a sensor probe 240. In some examples, the sensor probe 140 may be provided independently from the ablation probe assembly 106. The sensor probe 240 may be positioned at or near the target tissue in the patient during an ablation treatment to monitor and/or determine one or more parameters. For example, the sensor probe 240 may include one or more measurement points that can measure an impedance, temperature, moisture, density, or other characteristics of the patient. This measurement information can be transferred via a sensor cable 242 to the console 102. The sensor cable 242 may terminate at a sensor connector that can be connected to the console 102 at the first sensor port 220 and/or the first sensor port 222. This information can be sent to a processor, memory, or other element in the console 102 to be stored and/or displayed on the display 208. While the example base unit 102, include two sensor ports 224, 226, it should be appreciated that the console 102 may include less than two sensor ports or more than two sensor ports in other examples.

The console 102 may also include two or more coolant flow generators. The coolant flow generators may be configured to move coolant through the ablation probe assembly 106 to collect, transfer, and/or move thermal energy and prevent elements of the ablation probe assembly 106 from heating to undesirable levels. The coolant flow generators may each include a separate and independent pump that can be operated individually to cool the corresponding probe assembly 106. Various suitable pumps may be used such as a peristaltic pump.

The coolant flow generators may each be used through interaction with a corresponding coolant port. In the example shown, the console 102 includes a first coolant port 242 and a second coolant port 244. A complimentary cartridges 246 can be received in the first coolant port 242 and/or the second coolant port 244. In other examples, the coolant ports may be positioned in other regions of the console 102 such as in a side wall of the housing 204. The coolant cartridge 246 may include a cradle and tubing that permits the pump to interact with the coolant cartridge 246 to move coolant (e.g., saline) from the coolant receptacle 210 through the probe assembly 106. The coolant receptacle 210 can be supported on a hanger 212. The hanger 212 can be an upright hook or bar that suspends the coolant receptacle 210 in a position vertically above the coolant port of the console 102.

In the example shown, the console 102 includes two coolant flow generators. In other examples, the console 102 may include one coolant flow generator. In still other examples, the console 102 may include more than two coolant flow generators. The example shown in FIG. 2 also shows a single ablation probe assembly 106. It should be appreciated, however, that other ablation systems may include more than one ablation probe assembly 106. The number of ablation probe assemblies may correspond to the number of microwave generators, microwave ports, sensor ports, and/or coolant flow generators.

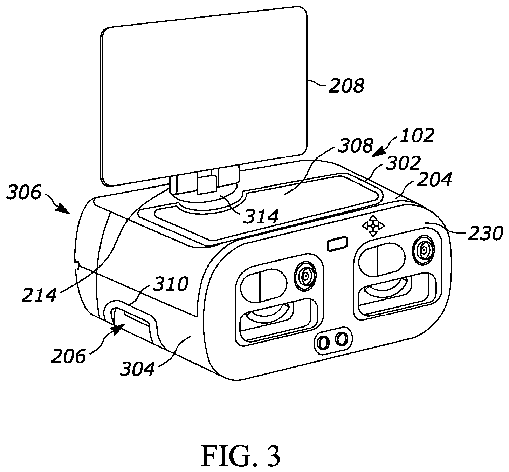

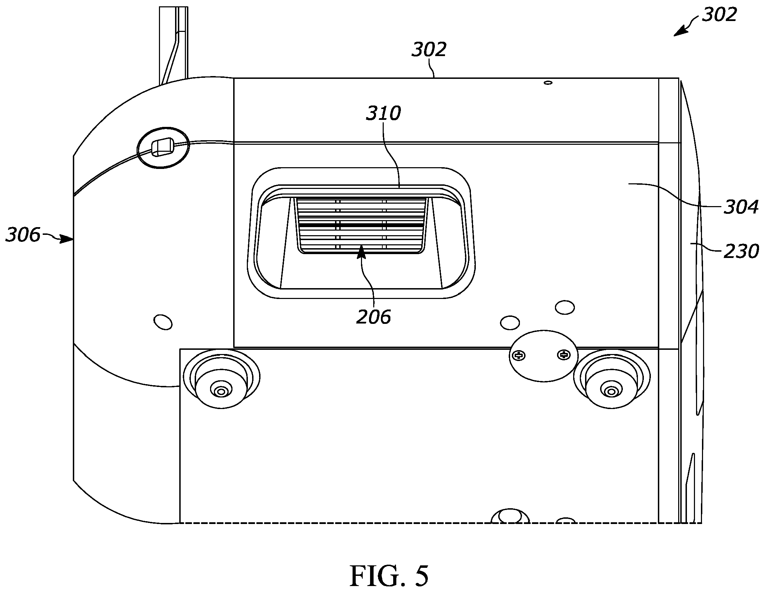

FIGs. 3-6 illustrate further aspects of one example housing 204 of the console 102. The housing 204 may be constructed of one more shell portions that are configured to mate together to form an outer structure of the console 102. The housing 204 may be configured to allow the console 102 to be easily moved and positioned in a desired position relative to a patient in a treatment environment. The housing 204 may also be constructed to improve durability and reduce a likelihood of damage to the console 102 and/or the internal components of the console 102.

The housing 204 may, in one example, include a front face 230, an upper shell portion 302, a lower shell portion 304, and a back portion 306. The front face 230, the upper shell portion 302, the lower shell portion 304, and the back portion 306 may be joined together to define an interior volume of the console 102 in which the internal components of the console 102 can be positioned. The lower shell portion 304 may include a lip and the upper shell portion 302 may include a complimentary flange that is positioned over the lip. This mating structure prevents liquids from entering the interior volume should a fluid be spilled or otherwise deposited on the console 102. The center region 308 of the upper shell portion 302 may have a depression or be positioned vertically lower than front and rear edges of the upper shell portion 302. This feature may guide fluids away from the back portion 306 and/or the front face 230. This can keep fluids from entering the connectors and ports that may be located on the back portion 306 and/or the front face 230. In addition, the depression located in the upper shell portion 302 may be sized to permit the display 208 to be nested inside the depression when the display 208 is folded to a flat position in which the display is positioned horizontally or in a position substantially parallel to an upper surface of the upper shell portion 302.

The housing 204 may also include one or more handles 310. In the example shown, the handles 310 may be integrally formed into the lower shell portion 304. In other examples, the handles 310 may be positioned elsewhere or attached using a suitable fastener or other attachment. One handle 310 may be positioned on one lateral side of the housing 302 and a second handle 310 may be positioned on an opposite lateral side of the housing 302. In such a configuration, a user may easily lift, move, and/or position the console 102 as desired.

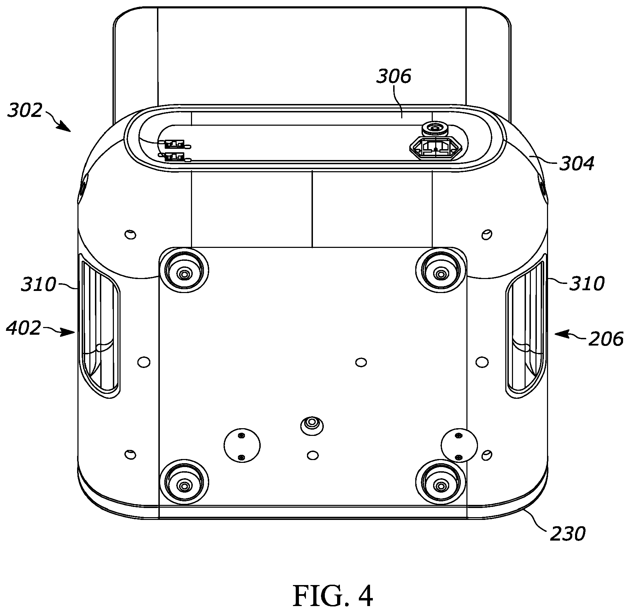

The housing 302 may also include an inlet opening 206 and outlet opening 402 (FIG. 4). The inlet opening 206 and the outlet opening 402 are formed in the lower shell portion 304 and are positioned on opposite lateral sides of the housing 302, in the example shown. The inlet opening 206 may be positioned and/or formed into the handle 310. The outlet opening 402 may be positioned and/or formed into the opposite handle 310. The inlet opening 206 may include slats, a grate, louvres, an array of holes or other suitable structure to allow air to flow into the inlet opening 206 while it may prevent larger items from passing into the interior volume of the console 102. In the example shown, the inlet opening 206 and the outlet opening 402 are each inset into the housing 302 or toward the interior of the housing 302. Such a configuration can reduce a likelihood that spills or other items can be dropped into the inlet opening 206 and/or the outlet opening 402.

The housing 204 may also include a hinge 214 and base 314 that may connect the display 208 to the upper shell portion 302. The hinge 214 can allow the display 208 to be rotated 360° relative to a top surface of the upper shell portion 302. The hinge 214 can also allow the display 208 to be tilted upwards and downwards to change an angle of the screen relative to the top surface of the upper shell portion 302. In a storage position, for example, the display 208 can be rotated downwards so that the screen of the display 208 is positioned substantially parallel to and adjacent the top surface of the upper shell portion 302. In a deployed position, the display can be rotated upwards and away from the top surface of the upper shell portion 302. The display 208 can be positioned, for example, substantially perpendicularly to the top surface of the upper shell portion 302.

The base 314 may allow the display 208 to be rotated relative to the upper shell portion 302. The viewable panel on the screen, for example, can be rotated to be viewed from multiple locations at or around the console 102. In some examples, the base 314 is rotatably coupled between the upper shell portion and the hinge 214. The base 314 may allow rotation of the display 360°, in some examples. In the example shown, the base 314 is mounted to the upper shell portion 302. In other examples, the rotatable base 314 may be positioned between the hinge 214 and the display 208. Using both the hinge 214 and the base 314, the display 208 can be rotated and angled in various suitable positions to allow viewing of the screen of the display 208 without the need to re-position or move the console 102.

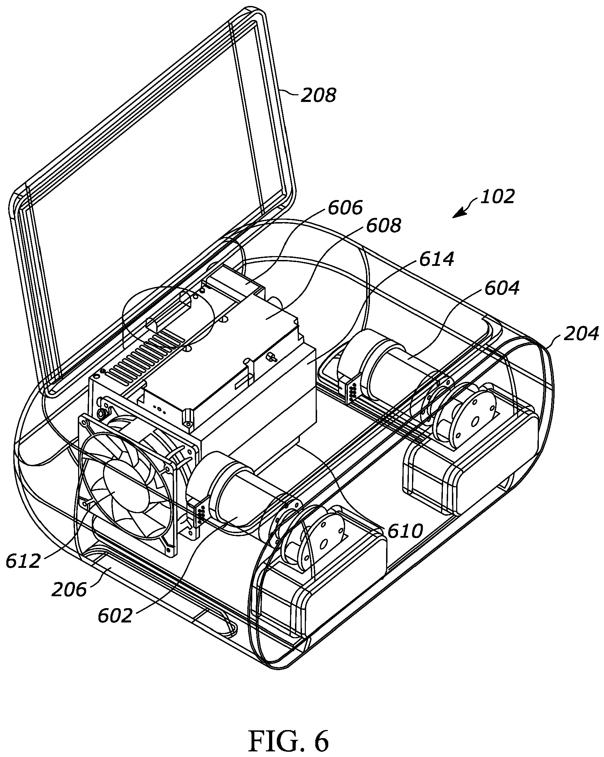

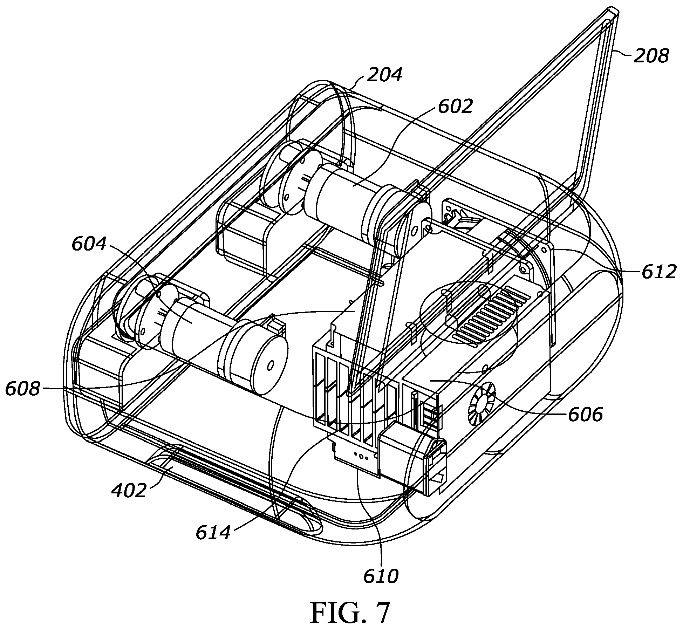

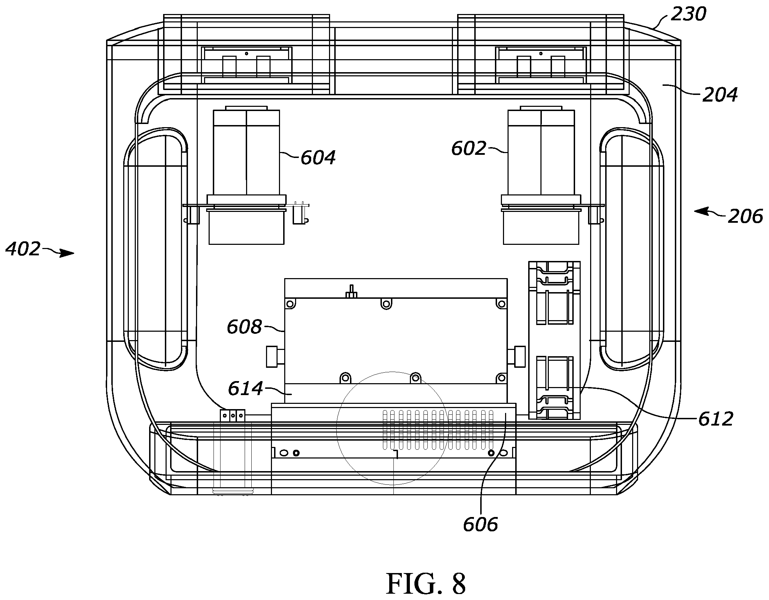

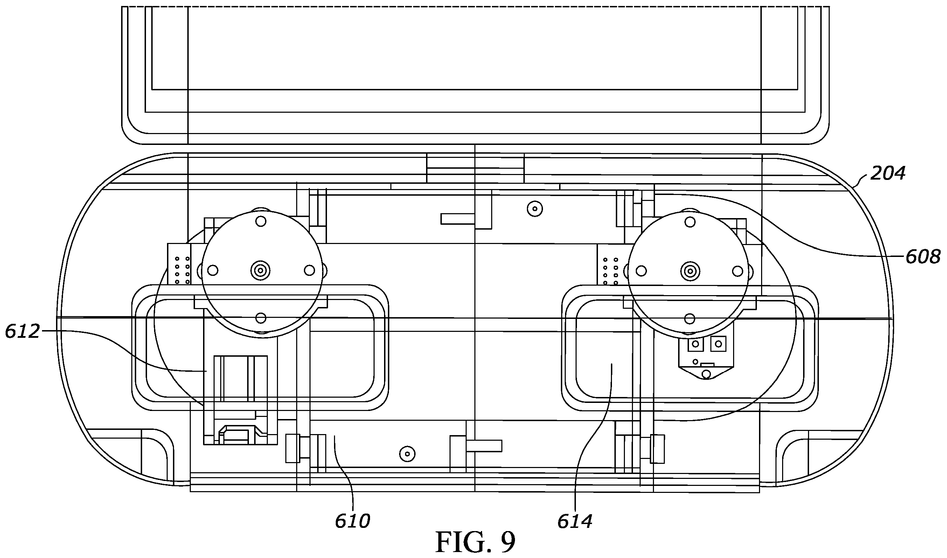

Referring now to FIGs. 6-9, the example console 102 is illustrated with the housing 204 transparent to show some internal components of the console 102. As shown, the housing 204 may enclose a first coolant generator or pump 602, a second coolant generator or pump 604, a power supply 606, a first microwave generator 608, a second microwave generator 610, a fan 612, and a heat sink 614. The console 102 may include other elements that are not shown for illustration purposes only. Such other elements may include, but are not limited to, a control unit, a printed circuit board (PCB), one or more air ducts, processors, memory, wiring, and/or other ablation components.

The first coolant generator 602 and the second coolant generator 604 may be positioned inside the interior volume of the housing 204 toward the front face 230. In this position, the first coolant generator 602 and the second coolant generator 604 may interface or be operable to interact with the first coolant port and the second coolant port previously described. The first coolant generator 602 and the second coolant generator 604 may be positioned in the interior volume of the housing 204 but spaced apart from the heat sink 614. As will be further described, the cooling system of the console 102 may operate to manage the thermal energy produced during operation of the first coolant generator 602 and/or the second coolant generator 604 even though the heat sink 614 may not be in direct contact with the first coolant generator 602 and the second coolant generator 604.

The heat sink 614 is positioned at a rear portion of the housing 204. The heat sink 614 may be in direct contact with one or more components of the console 102. As can be appreciated, it may be advantageous to position the heat sink 614 in direct thermal contact or in conductive contact with components of the console that generate significant amounts of thermal energy during use. The power supply 606, the first microwave generator 608, and the second microwave generator 610 may generate significant amounts of thermal energy during operation of the console 102. Without proper thermal management, the power supply 606, the first microwave generator 608, and/or the second microwave generator 610 may fail during use or prematurely.

The power supply 606 may be sized and configured to provide power to the multiple independent microwave generators that may be included in the console 102. In this example, the power supply 606 is sized and configured to provide power to both the first microwave generator 608 and the second microwave generator 610. The power supply 606 can be energized though the use of single power cord (not shown) that can be connected to an electrical outlet. This simplification allows a single input power source to provide power to multiple microwave generators. As can be appreciated, due to the size of the power supply 606 and the need to provide power to multiple microwave generators, the power supply 606 can generate significant amounts of thermal energy during operation. The power supply 606 therefore may be positioned in direct contact with the heat sink 614. In the example shown, the power supply 606 is connected to a back wall of the heat sink 614. In other examples, the power supply 606 may be connected at other locations relative to the heat sink 614. In examples, that include only a single microwave generator, the power supply 606 may still generate significant thermal energy. The positioning of the power supply 606 in such single generator examples may be similarly positioned.

The first microwave generator 608 and the second microwave generator 610 may also generate significant amounts of thermal energy during operation. The first microwave generator 608 and the second microwave generator 610 may each be connected to opposite sides of the heat sink 614. In the example shown, the first microwave generator 608 is connected in direct contact with a top wall of the heat sink 614 and the second microwave generator 610 is connected to a bottom wall of the heat sink 614. In other examples, the first microwave generator 608 and/or the second microwave generator 610 may be connected and/or positioned differently relative to the heat sink 614. In still other examples, the console 102 may include a single microwave generator or more than two generators. The positioning of the microwave generators (or single generator) may be similar to that shown. For example, in a single generator embodiment, the generator may positioned either at a similar position as the first microwave generator 608 and/or as the second microwave generator 610. In multiple generator embodiments, the multiple generators may be positioned along an elongated version of the heat sink 610 but in a similar manner as the first microwave generator 610 and the second microwave generator 614.

As will be further described, the heat sink 614 may be configured as an enclosure through which an air flow may be directed. The air flow may heat as it passes through the heat sink 614 to remove heat and cool the power supply 606, the first microwave generator 608, and/or the second microwave generator 610. The airflow may also be mixed with the air in the interior volume of the housing outside of the heat sink. The air flow may be moved out of the interior volume of the housing 204 to remove heat from the interior volume of the housing 204 to an external location.

The fan 612 may be connected at one end of the heat sink 614. In the example shown, the fan 612 is connected at an end of the heat sink 614 proximate the inlet opening 206. The fan 612 may be suitably arranged and operated to draw air into the interior volume of the housing 204 and move air through the heat sink 614. The air may then be moved to an external location after the air exits the housing 204 through the outlet opening 206.

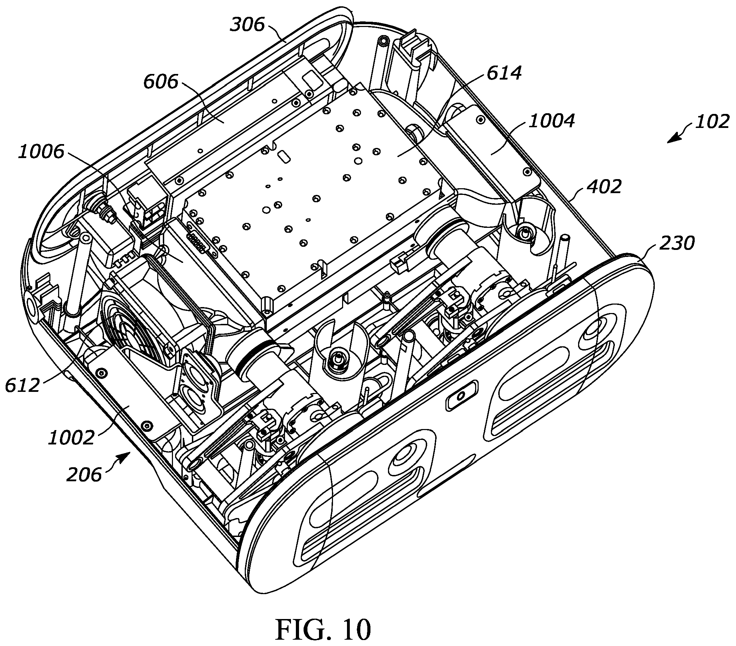

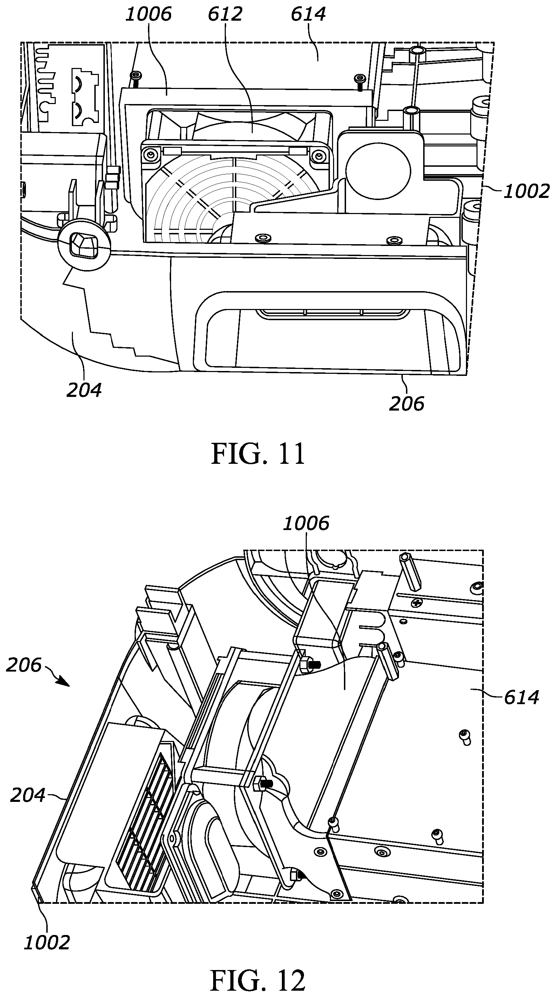

Turning now to FIG. 10, the example console 102 is illustrated with the upper shell portion 302 removed so as to expose interior volume of the housing 204. As can be seen, the console 102 may include an inlet duct 1002 that is shaped to guide air from the inlet opening 206 into the interior volume of the housing 204. The fan 612 may be offset from the inlet opening 206 and/or the inlet duct 1002. The fan 612, in this example, is positioned rearward or at least partially rearward of the inlet opening 206. As further shown in FIGs. 11 and 12, the inlet duct 1002 prevents air that enters the housing 204 from flowing directly through the fan 612 and into the heat sink 614. Instead, the inlet duct 1002 includes one or more walls that direct air upwards into the interior volume of the housing 204 adjacent to the heat sink 614. In this manner, the air that enters the housing 204 may mix with the existing air in the housing. This mixing can allow the air flow that is caused by the fan 612 to cool other internal components of the console 102 (e.g., coolant generators) in addition to those components in contact with the heat sink 614 (e.g., power supply and microwave generators).

The fan 612 can operate to direct air into heat sink 614. The fan 612 causes an air pressure gradient to be created that draws air into the housing 204 though the inlet opening 206. The air is directed into the interior volume of the housing 204 away from the heat sink 614. In the example shown, the inlet duct 1002 directs the air upwards into the housing 204. The fan 612 may then draw interior mixed air from inside the housing 204 into the heat sink 614. As the air moves through the heat sink 614, the air may be heated by drawing thermal energy away from the microwave generators and/or the power supply to keep these components at satisfactory operating temperatures. The heated air can then be moved to an external location outside the housing 204.

As further shown in this example, the fan 612 may be fluidly coupled to the heat sink 614 via a transition portion 1006. The transition portion 1006 may have an upstream side that is sized to couple to the fan 612 and a downstream side that is sized to couple to the heat sink 614. The upstream side and the downstream side of the transition portion 1006 may have different shapes because the fan 612 and the heat sink 614 may have different shapes. The transition portion 612 may be configured to cause substantially all of the air flow that enters the fan 612 to be moved to the heat sink 614. In different examples, the transition portion 1006 may have different shapes or may not be used if the fan 612 and the heat sink 614 have similar outer profiles or shapes.

The transition portion 1006 may also be aerodynamically shaped and/or contoured to minimize the loss of air pressure through the transition portion 1006 in order to optimize system airflow and cooling efficiency of the heat sink. The transition portion 1006 may include guide vanes, fins, baffles, or other features located in the interior to preferentially affect the airflow approaching or entering the heat sink. In one example, the transition portion 1006 may include interior features to aerodynamically minimize the loss of air pressure through the transition portion 1006 and/or to preferentially distribute the airflow density into and/or between different fluid plathways in the heat sink.

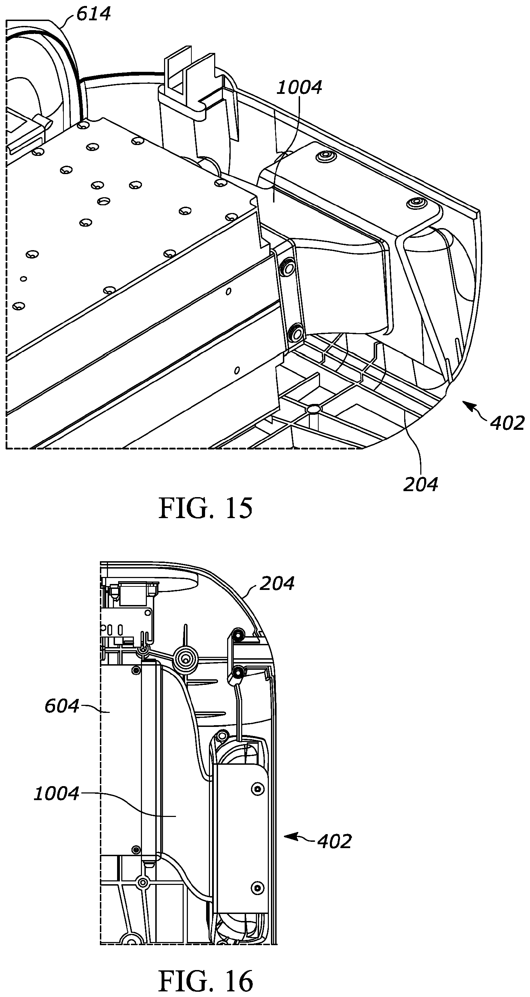

As shown in FIG. 10, the console 102 may include an outlet duct 1004. The outlet duct 1004 can be connected to an outlet end of the heat sink 614. The outlet duct 1004 may also be connected to the outlet opening 402. The outlet duct 1004 can direct air from the heat sink 614 out the outlet opening 402. In the example shown in FIGs. 15 and 16, the outlet duct 1004 may guide the air flow forward in the housing 204 to the outlet opening 402 since the heat sink 614 may be positioned rearward of the outlet opening 402. The outlet duct 1004 may guide substantially all of the air flow from the heat sink 614 to the outlet opening 402. It can be desirable to move the airflow from the heat sink to an external location because the air has been significantly heated due to a transfer of thermal energy from the microwave generators and the power supply into the air flow. Thus, it may be advantageous to direct the outlet air to an external location rather than allowing the outlet air to mix with air inside the housing 204 and/or to contact other internal components of the console 102.

While not shown, the heat sink 614 may include one or more fins that extend between or project from one or more walls of the heat sink into the interior of the heat sink 614. The fins may extend from a wall to conduct thermal energy from the microwave generator and/or the power supply that may be connected to the corresponding wall. For example, the heat sink 614 may include interior fins that extend horizontally between a front and rear wall of the heat sink 614. In other examples or in other portions of the heat sink 614, fins may extend vertically from a top wall or from a bottom wall of the heat sink 614. The fins may increase the surface area of the heat sink 614 to allow increased amounts of thermal energy to be transferred from the fins to the air flow that is moving over and around the fins.

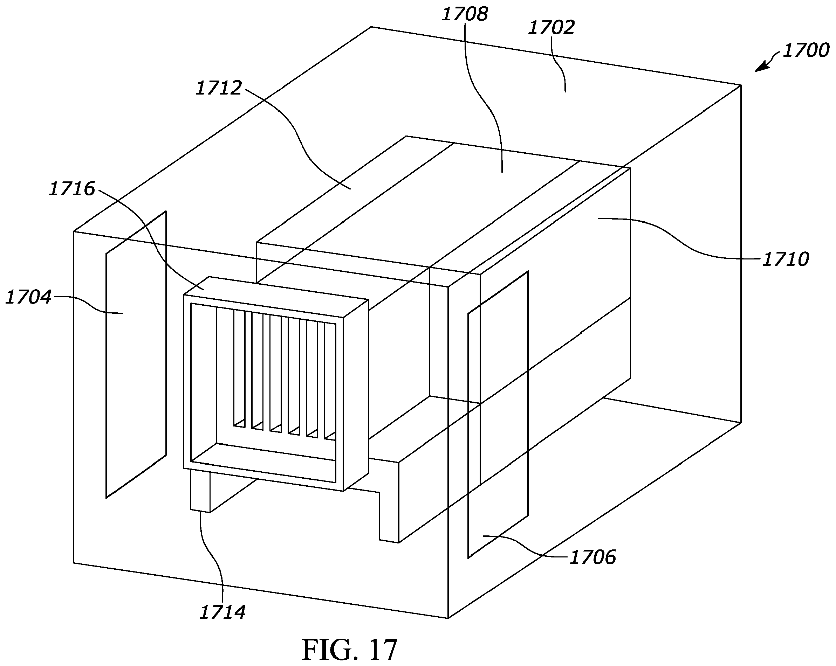

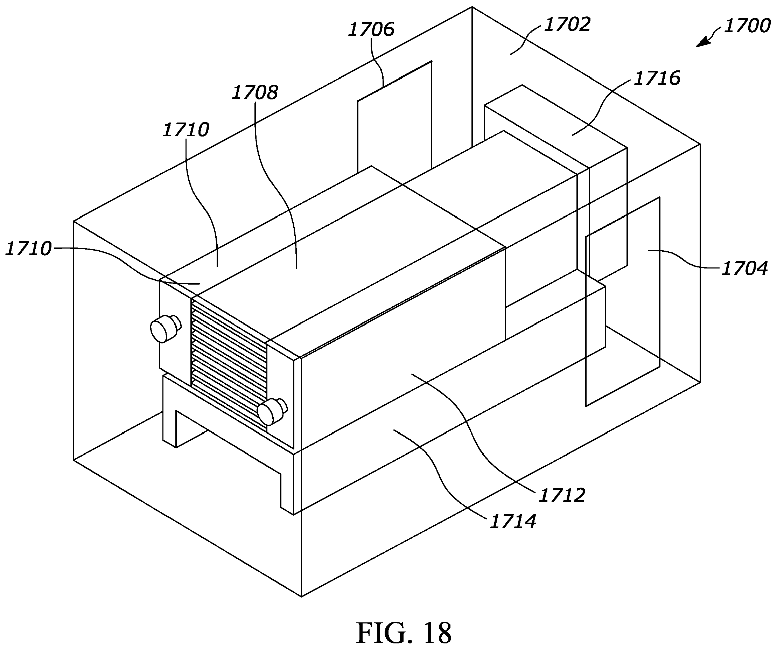

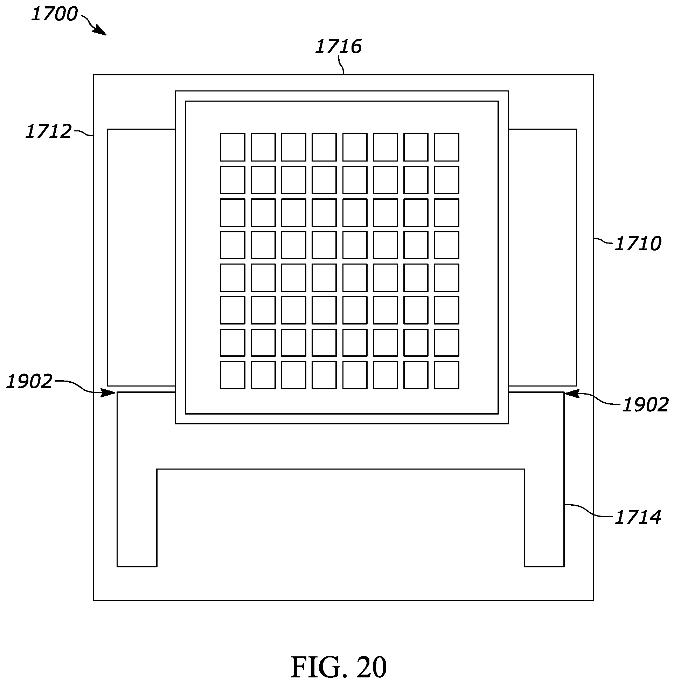

Referring now to FIGs. 17-21, another example cooling system is illustrated. In this example, console 1700 may include one or more walls to define an interior volume. While not shown, the console 1700 may be configured as a microwave ablation console and may include the features of console 102 previously described. In this illustration, the console 1700 includes a housing 1702. The housing 1702 encloses a first microwave generator 1710, a second microwave generator 1712, a power supply 1714, a heat sink 1708, and a fan 1716. The housing 702 may include a first opening 1704 and a second opening 1706. The first opening 1704 and the second opening 1706 may allow air to flow into the housing 1702 or out of the housing 1702. The fan 1716 may operate to cause air to be moved in or through the heat sink 1708 to manage the temperature of the first microwave generator 1710, the second microwave generator 1712, and/or the power supply 1714.

In this example, the fan 1716 may be positioned at an outlet end of the heat sink 1708. The fan 1716 may draw air into the heat sink 1708 through an end of the heat sink 1708 opposite to the fan 1716. In other examples, the fan 1716 may be positioned at an inlet end of the heat sink 1708 as previously described.

The first microwave generator 1710 and the second microwave generator 1712 may be positioned on opposite sides of the heat sink 1708. In this example, the first microwave generator 1710 is positioned on a lateral side of the heat sink 1708 and the second microwave generator 1712 is positioned on an opposite lateral side of the heat sink 1708. The power supply 1714, in this example, is positioned on a bottom side of the heat sink 1708.

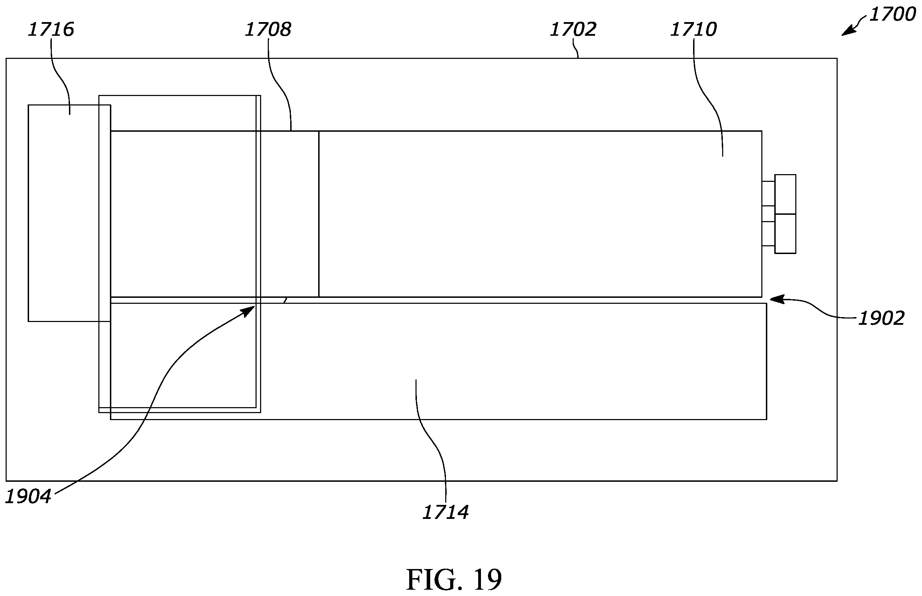

As shown in the side view of FIG. 19, the power supply 1714 may be spaced apart from the first microwave generator 1710 and from the heat sink 1708. As can be seen, the power supply 1708 is spaced from the first microwave generator 1710 and the heat sink 1708 by a gap 1902. At a longitudinal position downstream of the first microwave generator 1710, the power supply 1710 may contact the heat sink 1708 at position 1904. In this configuration, thermal conduction does not occur between the heat sink 1708 and the power supply 1714 until a position downstream of the microwave generators.

Such a configuration is desirable so that the air that flows through the heat sink (in a direction from right to left in FIG. 19) first collects thermal energy from the first microwave generator 1710 and the second microwave generator 1712. The air then moves to the portion of the heat sink 1708 downstream of the microwave generators (at or around position 1904) where thermal energy is collected from the power supply 1714. In this configuration, the microwave generators are cooled first so that they are maintained at sufficient operating temperatures since the microwave generators may be more susceptible to failure at elevated operating temperatures.

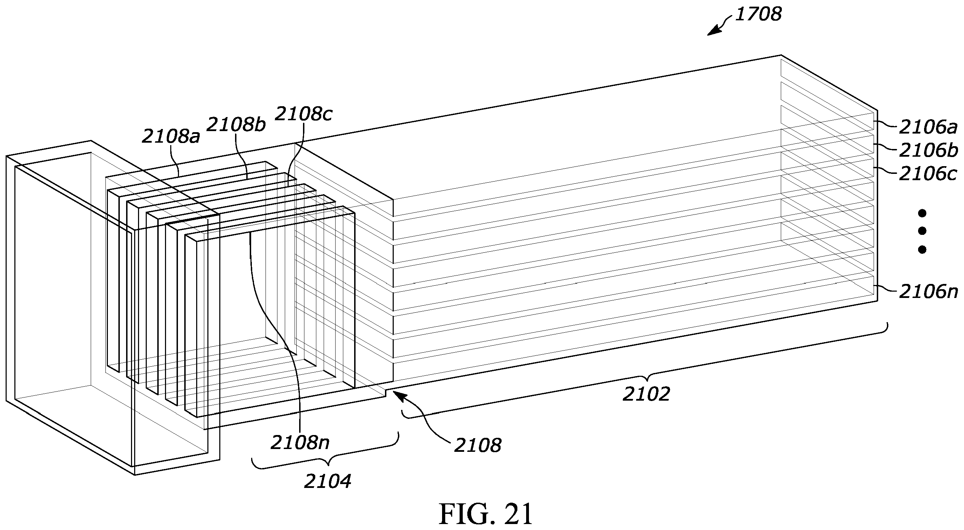

Referring now to FIG. 21, an example heat sink 1708 is shown. FIG. 21 shows the outer walls of the heat sink as transparent for illustration purposes only. In various embodiments, the walls and fins of the heat sink 1708 can be made of suitable materials having thermal conductivity properties to efficiently transfer thermal energy from the microwave generators and/or the power supply to the air moving through the heat sink 1708. Example materials include copper and aluminum. In other examples, other alloys or ceramics may also be used.

As previously described, the heat sink 1708 may include a microwave generator portion 2102 and a power supply portion 2104. The microwave generator portion 2102 may be located upstream of the power supply portion 2104. This orientation may allow the air moving through the heat sink 1708 to first be heated from thermal energy from the microwave generators before the air is further heated from thermal energy of the power supply. The heat sink 1708 may include a step 2108 to maintain a gap between the power supply and the heat sink 1708 at the microwave generator portion 2102.

The heat sink 1708 may include fins 2106, 2108 that extend across the internal cavity of the heat sink 1708. The fins 2106, 2108 are configured of the same or a similar material as the walls of the heat sink 1708 to conduct thermal energy from the microwave generators and the power supply to the air moving through the heat sink. The fins 2106, 2108 may be continuous layers of material that extend from one wall of the heat sink 1708 to an opposite wall of the heat sink 1708.

The fins 2106 may be positioned in the generator portion 2102 and the fins 2108 may be positioned in the power supply portion 2104. As shown, the fins 2106 in the generator portion may extend between the two opposite lateral walls of the heat sink 1708. In such an orientation, each of the fins 2106 may be oriented in a substantially horizontal plane and each fin 2106 may be oriented parallel to other fins 2106. The fins 2106 in the generator portion 2102 may extend between the two lateral walls since one generator is mounted to one lateral wall and a second generator is mounted to the opposite lateral wall. Thus, the fins 2106 extend between the walls of the heat sink 1708 to which the generators are mounted. In this manner, the thermal energy from the first generator and/or the second generator can be conducted from the wall of the heat sink through the fins 2106 to the interior of the heat sink 1708. As the air passes over the fins 2106, thermal energy is transferred from the first generator and/or the second generator to the air moving through the heat sink 1708 past fins 2106.

The fins 2108 in the power supply portion 2104 can be oriented in a different direction than the fins 2106. Since the power supply may be mounted to the bottom wall of the heat sink at the power supply portion 2104, the fins 2108 may extend in a substantially vertical plane and each fin 2106 can be oriented parallel to adjacent fins 2108. The thermal energy from the power supply can be transferred from the bottom wall of the power supply portion 2104 to the fins 2108 extending upward from the power supply. The air (after passing through the generator portion 2102) can pass through the power supply portion 2104. As the air passes the fins 2104, the thermal energy from the power supply can be transferred to the air via conduction through the bottom wall of the power supply portion 2104 and fins 2108 to the air flow.

It should be appreciated that the heat sink 1708 and/or aspects of the heat sink 1708 may be included in the heat sink 614 of the console 102 previously described. The heat sink 1708 may be included in the console 102 or other ablation consoles of the present disclosure. In addition, aspects of the console 102 and/or the heat sink 614 may be included or used in the heat sink 1708.

The example methods and apparatuses described herein may be at least partially embodied in the form of computer-implemented processes and apparatus for practicing those processes and/or the described functionality. The disclosed methods may also be at least partially embodied in the form of tangible, non-transient machine readable storage media encoded with computer program code. The media may include, for example, RAMs, ROMs, CD-ROMs, DVD-ROMs, BD-ROMs, hard disk drives, flash memories, or any other non-transient machine-readable storage medium, or any combination of these mediums, wherein, when the computer program code is loaded into and executed by a computer, the computer becomes an apparatus for practicing the method. The methods may also be at least partially embodied in the form of a computer into which computer program code is loaded and/or executed, such that, the computer becomes an apparatus for practicing the methods. When implemented on a general-purpose processor, the computer program code segments configure the processor to create specific logic circuits. The methods may alternatively be at least partially embodied in a digital signal processor formed of application specific integrated circuits for performing the methods.

The foregoing description of the embodiments has been provided for purposes of illustration and description. It is not intended to be exhaustive or to limit the disclosure. Individual elements or features of a particular embodiment are generally not limited to that particular embodiment, but, where applicable, are interchangeable and can be used in a selected embodiment, even if not specifically shown or described. The same may also be varied in many ways. Such variations are not to be regarded as a departure from the disclosure, and all such modifications are intended to be included within the scope of the disclosure.

## Claims

1. An apparatus for generating a microwave signal comprising:
a housing (204) comprising a plurality of walls defining an interior volume, an inlet opening and an outlet opening;
a power supply (606) positioned in the interior volume;
a first microwave generator (608) and a second microwave generator (610) each operably coupled to the power supply and each positioned in the interior volume spaced apart from the power supply;
a heat sink (614) positioned in the interior volume and contacting the power supply, the first microwave generator and the second microwave generator; and
a fan (612) positioned in the interior volume at a first end of the heat sink, the fan being configured to draw an airflow into the interior volume from the inlet opening and move the airflow through the heat sink wherein
the housing comprises a hinge configured to support and allow angular movement of a display relative to the housing.

2. The apparatus of claim 1, wherein the fan is offset from an inlet duct that is coupled to the inlet opening.

3. The apparatus of claim 1 or claim 2, wherein the housing comprises a rotatable base configured to allow rotational movement of the display relative to the housing.

4. The apparatus of any preceding claim, wherein the first generator and the second generator are positioned on opposite sides of the heat sink, and/or wherein the heat sink comprises a plurality of walls defining a chamber and a plurality of fins are positioned in the chamber, and/or wherein
the apparatus further comprises an outlet duct coupled to a second end of the heat sink opposite to the first end, wherein heated airflow from the heat sink is moved out the outlet opening through the outlet duct, and/or wherein the apparatus further comprises a plurality of coolant pumps positioned in the interior volume spaced apart from the heat sink.

5. The apparatus of any preceding claim, wherein at least a portion of the airflow that enters the interior volume through the inlet opening contacts internal components in the interior volume before entering the heat sink.

6. The apparatus of any preceding claim, further comprising an inlet duct coupled to the inlet opening, the inlet duct guiding incoming air upwards into the interior volume.

7. The apparatus of any preceding claim, wherein the housing comprises a first shell portion and a second shell portion, the first shell portion having a lip positioned around an outer circumference that is configured to overlap an outer circumference of the second shell portion, and/or wherein the housing comprises a first handle that projects inward from an outer surface of the housing, the inlet opening positioned in the first handle.

8. The apparatus of claim 7, wherein the housing comprises a second handle that projects inward from the outer surface of the housing, the outlet opening being positioned in the second handle, preferably wherein the first handle and the second handle are positioned on opposite sides of the housing.

9. The apparatus of any preceding claim, further comprising a stand connected to the housing, the stand being configured to support a coolant receptacle in a predetermined position relative to the housing.

10. The apparatus of any preceding claim, wherein the first microwave generator and the second microwave generator are both powered by the power supply.

11. The apparatus of any preceding claim, wherein the first microwave generator and the second microwave generator are independently operable to deliver a first microwave signal and a second microwave signal, respectively, optionally wherein the first microwave signal and the second microwave signal are different.

12. The apparatus of any preceding claim, wherein the power supply comprises a single power input.

## Patentansprüche

1. Vorrichtung zum Erzeugen eines Mikrowellensignals, umfassend:
ein Gehäuse (204), das eine Mehrzahl von Wänden umfasst, die ein Innenvolumen, eine Einlassöffnung und eine Auslassöffnung definieren;
eine im Innenvolumen angeordnete Stromversorgung (606);
einen ersten Mikrowellengenerator (608) und einen zweiten Mikrowellengenerator (610), die jeweils mit der Stromversorgung wirkverbunden sind und jeweils im Innenvolumen in einem Abstand von der Stromversorgung angeordnet sind;
einen im Innenvolumen angeordneten Kühlkörper (614), der mit der Stromversorgung, dem ersten Mikrowellengenerator und dem zweiten Mikrowellengenerator in Kontakt steht; und
einen Lüfter (612), der im Innenvolumen an einem ersten Ende des Kühlkörpers angeordnet ist, wobei der Lüfter dazu konfiguriert ist, einen Luftstrom von der Einlassöffnung in das Innenvolumen anzusaugen und den Luftstrom durch den Kühlkörper zu leiten,
wobei
das Gehäuse ein Scharnier umfasst, das dazu konfiguriert ist, eine Anzeige zu stützen und eine Winkelbewegung der Anzeige relativ zu dem Gehäuse zu ermöglichen.

2. Vorrichtung nach Anspruch 1, wobei der Lüfter gegenüber einem Einlasskanal versetzt ist, der mit der Einlassöffnung gekoppelt ist.

3. Vorrichtung nach Anspruch 1 oder 2, wobei das Gehäuse einen drehbaren Sockel umfasst, der dazu konfiguriert ist, eine Drehbewegung der Anzeige relativ zu dem Gehäuse zu ermöglichen.

4. Vorrichtung nach einem der vorhergehenden Ansprüche, wobei der erste Generator und der zweite Generator auf gegenüberliegenden Seiten des Kühlkörpers angeordnet sind, und/oder wobei der Kühlkörper eine Mehrzahl von Wänden umfasst, die eine Kammer definieren, und eine Mehrzahl von Kühlrippen in der Kammer angeordnet ist, und/oder wobei
die Vorrichtung ferner einen Auslasskanal umfasst, der mit einem zweiten Ende des Kühlkörpers, das dem ersten Ende gegenüberliegt, gekoppelt ist, wobei erwärmter Luftstrom von dem Kühlkörper durch den Auslasskanal aus der Auslassöffnung heraus bewegt wird, und/oder wobei die Vorrichtung ferner eine Mehrzahl von Kühlmittelpumpen umfasst, die im Innenvolumen in einem Abstand von dem Kühlkörper angeordnet sind.

5. Vorrichtung nach einem der vorhergehenden Ansprüche, wobei mindestens ein Teil des Luftstroms, der durch die Einlassöffnung in das Innenvolumen eintritt, mit internen Bauteilen in dem Innenvolumen in Kontakt kommt, bevor er in den Kühlkörper eintritt.

6. Vorrichtung nach einem der vorhergehenden Ansprüche, ferner umfassend einen Einlasskanal, der mit der Einlassöffnung gekoppelt ist, wobei der Einlasskanal einströmende Luft nach oben in das Innenvolumen leitet.

7. Vorrichtung nach einem der vorhergehenden Ansprüche, wobei das Gehäuse einen ersten Schalenabschnitt und einen zweiten Schalenabschnitt umfasst, wobei der erste Schalenabschnitt eine Lippe aufweist, die um einen Außenumfang herum angeordnet und dazu konfiguriert ist, einen Außenumfang des zweiten Schalenabschnitts zu überlappen, und/oder wobei das Gehäuse einen ersten Griff umfasst, der von einer Außenfläche des Gehäuses nach innen ragt, wobei die Einlassöffnung in dem ersten Griff angeordnet ist.

8. Vorrichtung nach Anspruch 7, wobei das Gehäuse einen zweiten Griff umfasst, der von der Außenfläche des Gehäuses nach innen ragt, wobei die Auslassöffnung in dem zweiten Griff angeordnet ist, vorzugsweise wobei der erste Griff und der zweite Griff auf gegenüberliegenden Seiten des Gehäuses angeordnet sind.

9. Vorrichtung nach einem der vorhergehenden Ansprüche, ferner umfassend einen mit dem Gehäuse verbundenen Ständer, wobei der Ständer dazu konfiguriert ist, einen Kühlmittelbehälter in einer vorbestimmten Position relativ zu dem Gehäuse zu tragen.

10. Vorrichtung nach einem der vorhergehenden Ansprüche, wobei der erste Mikrowellengenerator und der zweite Mikrowellengenerator beide von der Stromversorgung gespeist werden.

11. Vorrichtung nach einem der vorhergehenden Ansprüche, wobei der erste Mikrowellengenerator und der zweite Mikrowellengenerator unabhängig voneinander betreibbar sind, um ein erstes Mikrowellensignal beziehungsweise ein zweites Mikrowellensignal zu liefern, wobei optional das erste Mikrowellensignal und das zweite Mikrowellensignal verschieden sind.

12. Vorrichtung nach einem der vorhergehenden Ansprüche, wobei die Stromversorgung einen einzigen Stromeingang umfasst.

## Revendications

1. Appareil pour générer un signal micro-ondes comprenant :
un boîtier (204) comprenant une pluralité de parois définissant un volume intérieur, une ouverture d'entrée et une ouverture de sortie ;
une alimentation électrique (606) positionnée dans le volume intérieur ;
un premier générateur de micro-ondes (608) et un second générateur de micro-ondes (610), chacun couplé de manière fonctionnelle à l'alimentation électrique et positionné dans le volume intérieur à distance de l'alimentation électrique ;
un dissipateur thermique (614) positionné dans le volume intérieur et en contact avec l'alimentation électrique, le premier générateur de micro-ondes et le second générateur de micro-ondes ; et
un ventilateur (612) positionné dans le volume intérieur à une première extrémité du dissipateur thermique, le ventilateur étant configuré pour aspirer un flux d'air dans le volume intérieur à partir de l'ouverture d'entrée et déplacer le flux d'air à travers le dissipateur thermique, dans lequel le boîtier comprend une charnière configurée pour supporter et permettre le mouvement angulaire d'un écran par rapport au boîtier.

2. Appareil selon la revendication 1, dans lequel le ventilateur est décalé par rapport à un conduit d'entrée qui est couplé à l'ouverture d'entrée.

3. Appareil selon la revendication 1 ou la revendication 2, dans lequel le boîtier comprend une base rotative configurée pour permettre un mouvement de rotation de l'écran par rapport au boîtier.

4. Appareil selon une quelconque revendication précédente, dans lequel le premier générateur et le second générateur sont positionnés de part et d'autre du dissipateur thermique, et/ou dans lequel le dissipateur thermique comprend une pluralité de parois définissant une chambre et une pluralité d'ailettes sont positionnées dans la chambre, et/ou dans lequel l'appareil comprend en outre un conduit de sortie relié à une seconde extrémité du dissipateur thermique opposée à la première extrémité, dans lequel le flux d'air chaud provenant du dissipateur thermique est évacué par l'ouverture de sortie à travers le conduit de sortie, et/ou dans lequel l'appareil comprend en outre une pluralité de pompes à liquide de refroidissement positionnées dans le volume intérieur à distance du dissipateur thermique.

5. Appareil selon une quelconque revendication précédente, dans lequel au moins une partie du flux d'air qui pénètre dans le volume intérieur par l'ouverture d'entrée entre en contact avec les composants internes du volume intérieur avant d'entrer dans le dissipateur thermique.

6. Appareil selon une quelconque revendication précédente, comprenant en outre un conduit d'entrée couplé à l'ouverture d'entrée, le conduit d'entrée guidant l'air entrant vers le haut dans le volume intérieur.

7. Appareil selon une quelconque revendication précédente, dans lequel le boîtier comprend une première partie de coque et une seconde partie de coque, la première partie de coque ayant un rebord positionné autour d'une circonférence extérieure configurée pour chevaucher une circonférence extérieure de la seconde partie de coque, et/ou dans lequel le boîtier comprend une première poignée qui fait saillie vers l'intérieur à partir d'une surface extérieure du boîtier, l'ouverture d'entrée étant positionnée dans la première poignée.

8. Appareil selon la revendication 7, dans lequel le boîtier comprend une seconde poignée qui fait saillie vers l'intérieur de la surface extérieure du boîtier, l'ouverture de sortie étant positionnée dans la seconde poignée, de préférence dans lequel la première poignée et la seconde poignée sont positionnées de part et d'autre du boîtier.

9. Appareil selon une quelconque revendication précédente, comprenant en outre un support relié au boîtier, le support étant configuré pour supporter un réceptacle de liquide de refroidissement dans une position prédéterminée par rapport au boîtier.

10. Appareil selon une quelconque revendication précédente, dans lequel le premier générateur de micro-ondes et le second générateur de micro-ondes sont tous deux alimentés par l'alimentation électrique.

11. Appareil selon une quelconque revendication précédente, dans lequel le premier générateur de micro-ondes et le second générateur de micro-ondes fonctionnent indépendamment pour délivrer respectivement un premier signal micro-ondes et un second signal micro-ondes, éventuellement dans lequel le premier signal micro-ondes et le second signal micro-ondes sont différents.

12. Appareil selon une quelconque revendication précédente, dans lequel l'alimentation électrique comprend une seule entrée d'alimentation.
